# EUROPEAN PATENT SPECIFICATION

(11) **EP 4 586 911 B1**
(45) Date of publication and mention of the grant of the patent: **18.11.2026**
(21) Application number: 23866159.9
(22) Date of filing: 13.09.2023
(51) Int. Cl.: A61B 5/1455, C12Q 1/54, G01N 21/78, C12Q 1/26, G01N 33/58, A61B 5/157, G01N 21/75

(54) **LUMINESCENT ENZYME-BASED SENSORS**
LUMINESZENZSENSOREN AUF ENZYMBASIS
CAPTEURS À BASE D'ENZYMES À LUMINESCENCE

(30) Priority: 14.09.2022 US 202263406567 P
(43) Date of publication of application: 23.07.2025
(73) Proprietor: Terumo Cardiovascular Systems Corporation, Ann Arbor, Michigan 48103 (US)
(72) Inventor: BAEUMNER, Antje J., 93053 Regensburg (DE); DUERKOP, Axel, 93053 Regensburg (DE); BAUER, Meike, 93053 Regensburg (DE); GROTZ, Barbara Veronika, 93053 Regensburg (DE); GALLIGAN, John Josef, 93053 Regensburg (DE); RAJU, Liju Gheevarghese, Ann Arbor, Michigan 48103 (US)
(74) Representative: Fish & Richardson P.C.
(86) International application number: PCT/US2023/032647
(87) International publication number: WO 2024/059143

(56) References cited:
- US-A- 4 795 707
- US-A- 4 890 620
- US-A- 5 789 255
- US-A1- 2002 137 027
- US-A1- 2017 238 856
- US-A1- 2019 383 801
- US-A1- 2019 383 801
- WOLFBEIS O S ET AL: "RECENT PROGRESS IN OPTICAL OXYGEN SENSING", 1 January 1990, SELECTED PAPERS ON OPTICAL FIBERS IN MEDICINE; [SPIE MILESTONE SERIES], BELLINGHAM, SPIE, US, PAGE(S) 351 - 357, XP000467824

## Description

### BACKGROUND

### 1. Technical Field

This document relates to medical systems for sensing biological analytes using enzymes. For example, this document relates to sensors for the continuous monitoring of glucose and/or lactate in aqueous solutions and body fluids based on a readout of fluorescent or luminescent signals.

### 2. Background Information

Monitoring of biological analytes such as pH, blood gases, electrolytes, and metabolites has been one of the primary avenues to assess the general health of individuals and the status of their bodily functions, especially in critical-care settings. For example, dedicated analyzers are used in near-patient testing environments to provide for continuous, real-time measurement and detection of blood analytes during critical care situations. Measurement of blood analytes provides valuable information regarding the state of oxygenation, gas exchange, acid-base homeostasis, and ventilation of an individual. Though various biological analyte sensor technologies have been developed, improvements in design, functionality, and accuracy are continually sought. Additionally, if incorrect or incompatible solvent or membrane materials are used, or if an interference barrier is improperly located, the enzymes and/or probes can lose functionality. Currently, no sensor exists that can provide real-time, continuous monitoring of biological analytes such as glucose and/or lactate in blood during surgeries or at bedside.

US 2002/0137027 describes biological methods of determination using enzymes from the group of oxidases and using optical indicators from the group of lanthanoid-ligand complexes.

US 2017/0238856 describes a continuous analyte sensor that can be used to measure glucose or lactate levels in a patient, along with other analytes. The sensor can be located in the tissue or a blood vessel of a patient, and a probe can be located on the skin of the patient generally adjacent to the sensor. The probe can detect luminescent signals that originate from the sensor and that are dependent on analyte levels.

US 2019/0383801 describes a glycemic management related analyte detecting assay device for detecting and quantifying analyte concentrations in a fluid sample. The assay device includes an absorbent body containing an assay forming a detection zone for receiving a fluid test sample. The absorbent body is provided in a chamber of the device. The assay can detect one or more of a glycemic analyte selected from the group consisting of fasting plasma blood glucose, oral glucose, % glycated hemoglobin, and fasting insulin concentrations.

### SUMMARY OF THE INVENTION

According to the invention, there is provided a flow-through blood parameter measurement device according to claim 1, preferred embodiments being specified in the dependent claims 2-15.

### SUMMARY OF THE DISCLOSURE

This document describes medical systems for sensing biological analytes using enzymes. For example, this document describes sensors for the continuous monitoring of biological analytes such as glucose and/or lactate in aqueous solutions and body fluids based on a readout of luminescence signals.

In one aspect, this disclosure is directed to a blood parameter measurement device as recited in claim 1.

In some cases, the enzyme is selected from the group consisting of: glucose oxidase (GOx), lactate oxidase (LOx), cholesterol oxidase (ChOx), galactose oxidase (GAOx), pyruvate oxidase (POx), xanthine oxidase (XAOx), monoamine oxidase A (MAO-Ax), monoamine oxidase B (MAO-Bx), D-Amino acid oxidase (D-AAOx), L-Amino acid oxidase (L-AAOx), lactose oxidase (LOx), and superoxide dismutase (SOD). In some cases, the enzyme is or comprises a glucose oxidase (GOx). In some cases, the enzyme is or comprises a lactate oxidase (LOx).

In some cases, the substance that is chemically responsive to hydrogen peroxide comprises a Europium(III)-tetracycline (EuTu) complex. In some cases, the first layer and the second layer are directly adjacent to each other.

In some cases, the sensor further comprises an intermediate layer between the first layer and the second layer. In some cases, the first layer is an annular layer defining an open space, and is substantially centered on the second layer. In some cases, the sensor further comprises a protective layer positioned between the first layer and the interior space.

In some cases, the sensor further comprises a reference dye. In some cases, the reference dye is selected from the group consisting of: 9,10 di(phenylenthynyl)anthracene (DPEA), CD405M, 1-anilinonaphthalene-8-sulfonic acid, 8-benzyloxy-5,7-diphenylquinoline, 4-methylumbelliferyl acetate, octadecyl 7-hydroxycoumarine-3-carboxylate, perylene, tetracene, H9-40, pyranine, etyl eosin, coumarin 30, coumarin 153, and CF^{™}405M. In some cases, the reference dye has an excitation maximum at 400±10 nm and an emission maximum at 450±10 nm. In some cases, the sensor further comprises a reference layer comprising the reference dye. The reference layer can be positioned farther from the interior space than the second layer.

The technology described in this document can provide one or more advantages and/or benefits. For example, this technology facilitates real-time monitoring of blood parameters, which provides critical information required for goal-directed perfusion during cardiopulmonary bypass surgery, and continuous analyte detection in blood via luminescence measurements. This technology also permits ongoing bedside monitoring of patient body fluids including, but not limited to, blood. The technology described is an affordable system that uses relatively inexpensive components and can take advantage of inexpensive mass production and/or roll-to-roll fabrication.

Unless otherwise defined, all technical and scientific terms used herein have the same meaning as commonly understood by one of ordinary skill in the art to which this invention pertains. Although methods and materials similar or equivalent to those described herein can be used, suitable methods and materials are described herein. In case of conflict, the present specification, including definitions, will control. In addition, the materials, methods, and examples are illustrative only and not intended to be limiting.

The details of one or more embodiments are set forth in the accompanying drawings and the description herein. Other features, objects, and advantages will be apparent from the description and drawings, and from the claims.

### DESCRIPTION OF THE DRAWINGS

FIG. 1 is a schematic diagram of a patient undergoing open-heart surgery while being supported using a heart-lung system and an extracorporeal circuit.
FIG. 2 is a perspective view of an example blood parameter measurement system in accordance with some embodiments.
FIG. 3 schematically depicts the multi-layered construction of an example sensor in accordance with some embodiments.
FIG. 4 schematically depicts the multi-layered construction of an example sensor in accordance with some embodiments.
FIG. 5 schematically depicts the multi-layered construction of an example section of a sensor in accordance with some embodiments.
FIG. 6 schematically depicts the multi-layered construction of an example sensor in accordance with some embodiments.
FIG. 7 schematically depicts the multi-layered construction of an example sensor in accordance with some embodiments.
FIG. 8 schematically depicts the multi-layered construction of an example sensor in accordance with some embodiments.
FIG. 9 is a flowchart of an example method for manufacturing the multi-layered sensor in accordance with some embodiments.
FIG. 10 is a flowchart of an example method for manufacturing the multi-layered sensor in accordance with some embodiments.
FIG. 11 is a schematically depicts using sensor measurement in a flow cell or flow-through format.
FIG. 12 is a schematically depicts the multi-layered construction of an example sensor in accordance with some embodiments

Like reference numbers represent corresponding parts throughout.

### DETAILED DESCRIPTION

This document describes medical systems and devices for sensing and/or measuring biological analytes. For example, this document describes optical sensors, in some cases enzyme-based sensors, for the continuous monitoring of biological analytes, for example, glucose and/or lactate, in aqueous solutions and body fluids (e.g., blood) based on a readout of luminescence or fluorescent signals.

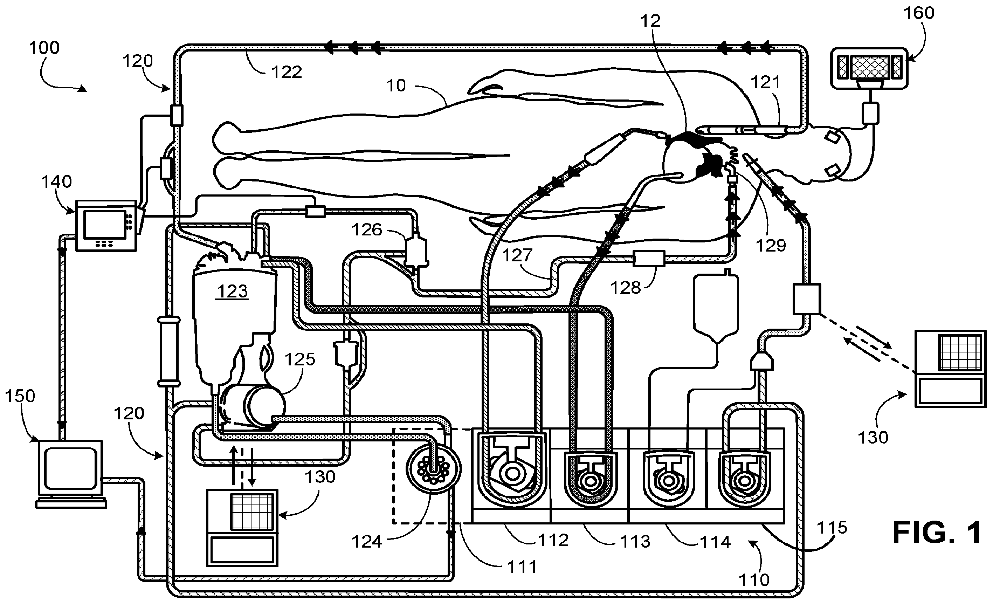

Referring to **FIG. 1****,** various types of medical procedures can be performed on a patient 10 while the patient 10 is connected to a life-sustaining heart-lung machine ("HLM") system 100. Before, during, and/or after such a procedure, parameters of the blood of the patient 10 can be measured to monitor the condition of the patient 10. As described further below, the types of patient parameters that can be measured include glucose and/or lactate in the blood of the patient 10.

In this example, the patient 10 is undergoing open-heart surgery during which the heart 12 and lungs of the patient 10 are temporarily intentionally caused to cease functioning. Because the body of the patient 10 continues to have a metabolic need to receive a supply of circulating oxygenated blood during the medical procedure, the HLM system 100 performs such functions. That is, the HLM system 100 is connected to the patient 10 and performs the functions of the heart 12 and lungs of the patient 10 so that the patient 10 stays alive and healthy during open-heart surgery. The types of procedures that can be performed on the patient 10 in the manner depicted include, but are not limited to coronary artery bypass grafts, heart valve repairs, heart valve replacements, heart transplants, lung transplants, ablation procedures, repair of septal defects, repair of congenital heart defects, repair of aneurysms, pulmonary endarterectomy, pulmonary thrombectomy, and the like.

In the depicted example, the HLM system 100 includes components and sub-systems such as a HLM 110, an extracorporeal circuit 120, one or more temperature control systems 130, a blood monitoring system 140 (e.g., a CDI^{®} Blood Parameter Monitoring System), a perfusion data management system 150, and a regional oximetry system 160. Some types of procedures that use the HLM system 100 may not require all of the components and sub-systems that are shown. Some types of procedures that use the HLM system 100 may require additional components and/or sub-systems that are not shown.

The extracorporeal circuit 120 is connected to the patient 10, and to the HLM 110. Other systems, such as the temperature control system 130, blood monitoring system 140, and perfusion data management system 150 may also be arranged to interface with the extracorporeal circuit 120. The extracorporeal circuit 120 is connected to the patient 10 at the patient's heart 12. Oxygen-depleted blood (venous blood) from the patient 10 is extracted from the patient 10 at the patient's heart 12 using a venous catheter 121. The blood is circulated through the extracorporeal circuit 120 to receive oxygen and remove carbon dioxide. The oxygenated blood is then returned through the extracorporeal circuit 120 to the patient's heart 12 via an aortic cannula 129.

Briefly, the extracorporeal circuit 120 operates by removing venous, oxygen-depleted blood from the patient 10 via the venous catheter 121, and depositing the venous blood in the reservoir 123 via the venous tube 122. Blood from the reservoir 123 is drawn from the reservoir 123 by the pump 124. While the depicted embodiment includes a one-time use centrifugal pump as the pump 124, in some cases a peristaltic pump of the HLM 110 is used instead. The pressure generated by the pump 124 propels the blood through the oxygenator 125. The now oxygen-rich arterial blood exits the oxygenator 125, travels through the arterial filter 126 to remove emboli, through the arterial tube 160 via the aortic cannula 129.

During a surgical procedure using the HLM system 100, various vital signs of the patient 10 are measured and/or monitored. For example, the HLM system 100, as depicted, includes the blood monitoring system 140. The blood monitoring system 140 uses one or more blood gas sensors (e.g., venous shunt sensor, arterial shunt sensor, H/S cuvette, etc.) located at various locations along the extracorporeal circuit 120 (e.g., the venous tube 122, the arterial tube 127, etc.) to monitor the priming solution, as well as the arterial and/or venous extracorporeal blood of the patient 10 during the surgical procedure. The blood monitoring system 140 can also use one or more other biological analyte (e.g., blood gas or blood metabolite) sensors, which can include an air detector, a bubble sensor, an arterial optical fluorescent sensor, a venous optical fluorescent sensor, an arterial optical reflective sensor, a venous optical reflective sensor, a hematocrit level sensor, and/or a hemoglobin sensor. Parameters being monitored can include, but are not limited to, pH, pCO₂, pO₂, K⁺, temperature, SO₂, hematocrit, hemoglobin, base excess, bicarbonate, oxygen consumption and oxygen delivery. The parameters being monitored in the blood of the patient 10 can also include other blood metabolites such as glucose and/or lactate in accordance with the disclosure provided herein. Additional and non-limiting blood metabolites being monitored in the blood of the patient 10 can also include cholesterol, galactose, pyruvate, xanthine, amines (e.g., dopamine, norepinephrine, and/or serotonin), benzylamine, phenethylamine, D-amino acids, L-amino acids, lactose, creatine, insulin, heparin, and/or superoxide radicals (O₂⁻). Moreover, in some cases the devices and systems described herein can be used to monitor substances such as cell cultivate solutions and organ preservation liquids (e.g., packed red blood cells, human albumin, succinylated gelatin, NaHCO₃, NaCl, Insulin, heaparin sodium (HeaparinNa), antibiotic, calcium gluconate, etc.).

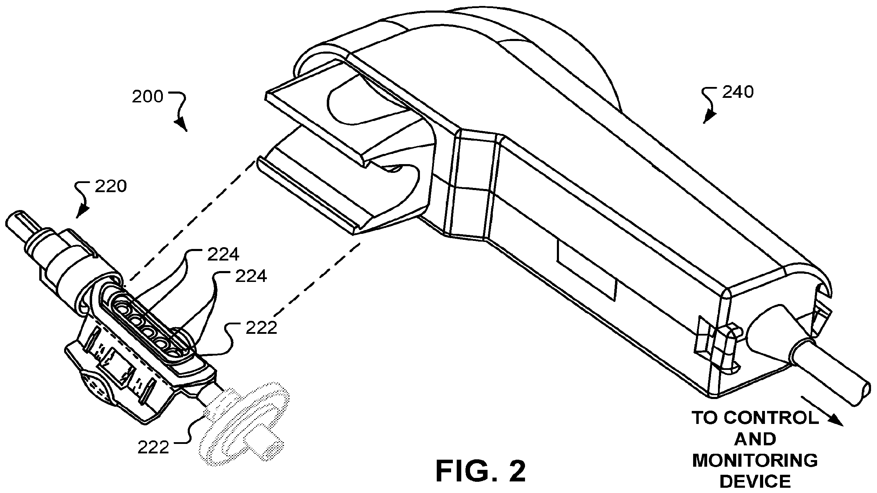

Referring to **FIG. 2****,** a blood parameter measurement system 200 (or simply "system 200"), especially useful for surgical procedures and/or for patient bedside monitoring, includes a shunt sensor 220 ("also referred to herein as a "blood parameter measurement device 220" or simply a "device 220") and an optical probe 240. The optical probe 240 is in wireless or wired communication with a control and monitoring device (not shown).

The blood parameter measurement device 220 and the optical probe 240 are releasably coupleable together. In the coupled configuration, the optical probe 240 operates in conjunction with the blood parameter measurement device 220 to measure various parameters of the body fluid that is within or flowing through the blood parameter measurement device 220.

In use, the blood parameter measurement device 220 is fluidly coupled with an extracorporeal source of body fluid (e.g., blood). The body fluid flows through the blood parameter measurement device 220. For example, in some cases one end of the blood parameter measurement device 220 is connected to a first tube that supplies blood to the blood parameter measurement device 220 and the other end of the blood parameter measurement device 220 is connected to second tube through which blood flows away from the blood parameter measurement device 220. Hence, the body fluid (e.g., blood) flows through the blood parameter measurement device 220.

The blood parameter measurement device 220 includes a tubular housing 222 to which one or more sensors 224 are attached. The tubular housing 222 defines an interior space configured for receiving blood.

The one or more sensors 224 are especially constructed to be responsive to one or more particular parameters of the body fluid (e.g., blood, etc.). The one or more sensors 224 each comprise a multi-layer assembly that can be adhesively attached to the tubular housing 222 of the blood parameter measurement device 220. In some cases, the adhesive used to adhesively attach the one or more sensors 224 to the tubular housing 222 can be pressure sensitive. The inner-most layer of the multi-layer assembly can come into direct contact with the bodily fluid within or flowing through the interior space of the blood parameter measurement device 220. In the example embodiment shown, a series of four sensors 224 are included as part of the blood parameter measurement device 220. For example, the sensors 224 can include an ion (potassium) sensor, a pH sensor, a carbon dioxide sensor and an oxygen sensor. Additionally, or optionally, the sensors 224 can also include one or more sensors for measuring additional biological analytes, such as metabolites, for example, glucose and/or lactate, in accordance with the disclosure provided herein.

In some embodiments, each of the one or more sensors 224 can optionally comprise a fluorescent ionophoric compound ("the ionophore") that contains a complexing moiety for binding an ion and a fluorescing moiety. The compound has a wavelength of maximum absorbance of at least about 350 nm. Suitable fluorescing moieties preferably contain close-lying nπ* and ππ* excited states. Suitable fluorescing moieties, when coupled to an appropriate complexing moiety, preferably are capable of ion dependent out-of-plane puckering. Also, the ππ* state of suitable fluorescing moieties preferably is sufficiently high in energy that ion dependent mixing dominates non-radiative coupling to the ground state. Particularly preferred fluorescing moieties include coumarin moieties, although other aromatic carbonyls or nitroaromatics or N-heterocyclic moieties may be employed. Suitable ion complexing moieties include cyclic "cage" moieties capable of binding an ion. The cage can be capable of selective binding of an ion. In some cases, preferred ion complexing moieties include crypt and crown ether moieties.

In some embodiments, the ionophore is covalently bonded to a suitable substrate that can be attached to the backing membrane. The substrate can be a polymeric material that is water-swellable and permeable to the ionic species of interest, and is preferably insoluble in the medium to be monitored. Particularly useful substrate polymers include, but are not limited to, ion-permeable cellulosic materials, high molecular weight or crosslinked polyvinyl alcohol (PVA), dextran, crosslinked dextran, polyurethanes, quaternized polystyrenes, sulfonated polystryrenes, polyacrylamides, polyhydroxyalkyl acrylates, polyvinyl pyrrolidones, hydrophilic polyamides, polyesters and mixtures thereof. In some embodiments, the substrate is cellulosic, especially ion-permeable crosslinked cellulose. In particular embodiments, the substrate comprises a regenerated cellulose membrane (Futamura P5-1 Membrane, Futamura Chemical-Manufacturer) that is crosslinked with an epoxide, such as butanediol diglycidyl ether, further reacted with a diamine to provide amine functionality pendant from the cellulosic polymer.

The optical probe 240 includes at least one light source that directs light toward the one or more sensors 224. Each of the one or more sensors 224 has a corresponding individual light source. The optical probe 240 also includes at least one light detector for detecting light emitted from the one or more sensors 224. Each of the one or more sensors 224 has a corresponding individual light detector. The system 200 includes a signal converter that is connected to the at least one light detector. The signal converter provides a digital output signal that varies in response to the quantity of light detected by each of the light detectors.

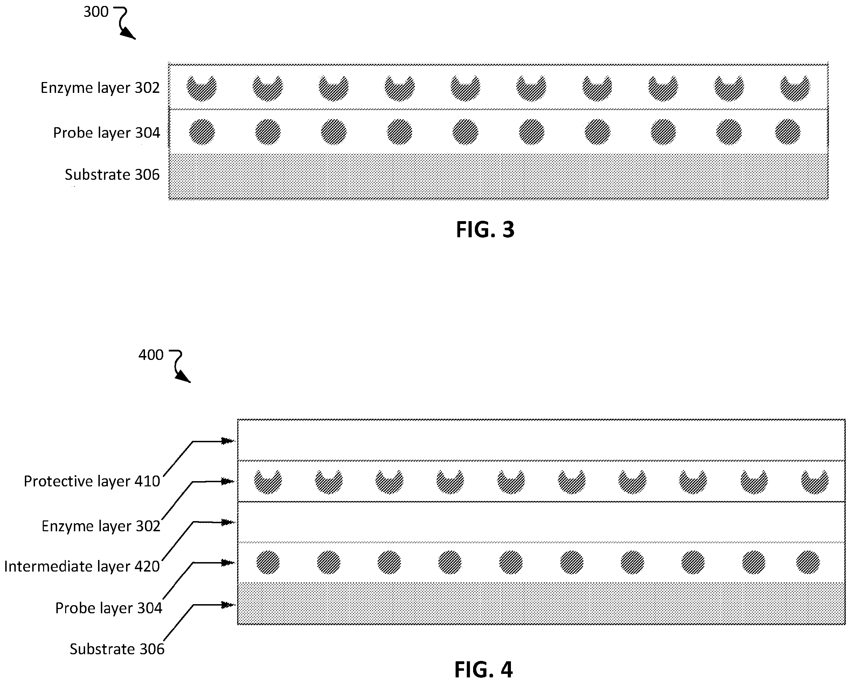

Referring to **FIG. 3****,** in some embodiments, a sensor 300 can include an enzyme layer 302, and a probe layer 304. The enzyme layer 302 is closer to the interior space than the probe layer 304. As such, the enzyme layer 302 is closer to the body fluid (e.g., blood), when the blood parameter measurement device 220 is filled with a blood or another body fluid. In some cases, the enzyme layer 302 is directly adjacent to the probe layer 304, and the enzyme layer 302 is closer to the interior space than the probe layer 304.

The enzyme layer 302 can be a hydrogel in which an enzyme is trapped or immobilized. The hydrogel can include an ether-based hydrophilic urethane, such as Hydromed^{™} D4, or a 4-(2-hydroxyethyl)-1-piperazineethanesulfonic acid (HEPES) and ethanol-water mixture. In some cases, the hydrogel can have a pH from about pH 7 to about pH 8 (e.g., about pH 7.4).

The biological analyte can diffuse from the bodily fluid to at least the enzyme layer 302. The enzyme can produce hydrogen peroxide when reacting with at least one biological analyte in body fluid (e.g., blood). The enzyme can be any enzyme that produces hydrogen peroxide. Such enzymes can include glucose oxidase (GOx), lactate oxidase (LOx), cholesterol oxidase (ChOx), galactose oxidase (GAOx), pyruvate oxidase (POx), xanthine oxidase (XAOx), monoamine oxidase A (MAO-Ax), monoamine oxidase B (MAO-Bx), D-Amino acid oxidase (D-AAOx), L-Amino acid oxidase (L-AAOx), lactose oxidase (LOx), and superoxide dismutase (SOD). In some cases, the enzyme comprises a glucose oxidase (GOx). In some cases, the enzyme comprises a lactate oxidase (LOx).

Non-limiting examples of biological analytes that can be measured with the blood parameter measurement device 220 are discussed above. In some cases, the biological analyte is glucose. In some cases, the biological analyte is lactate.

The hydrogen peroxide produced by the enzyme can diffuse at least to the probe layer 304. The probe layer 304 include a substance that is chemically responsive to hydrogen peroxide, such as the hydrogen peroxide produced by the enzyme in the enzyme layer 302. One example of the substance includes an Europium(III)-tetracycline complex (also known as an "EuTu" complex). A complex Europium(III)-tetracycline complex can coordinate with hydrogen peroxide at physiological relevant pH. This coordination can lead to an increase of luminescence intensity of the ⁵D₀→⁷F₂ transition of the Eu³⁺ ion, detectable at 616 nm. The light produced by the substance that is chemically responsive to hydrogen peroxide can be detected by, for example, an optical probe 240.

Additionally, in some embodiments the sensor can include one or more additional layers. One such layer is a substrate 306. Any appropriate substrate can be used. An appropriate substrate 306 allows for transmission of light from 350nm - 800nm. Non-limiting examples of a substrate 306 can include polyethylene terephthalate (PET) film, such as biaxial orientated PET film/foil (e.g., Mylar^{®}), and polycarbonate. The substrate 306 can be coated with an adhesive (e.g., a pressure sensitive adhesive). In some embodiments, the substrate 306 can be from about 100 µm to about 150 µm thick (e.g., from 110 µm to about 140 µm, or about 120 µm to about 130 µm). In some embodiments, the substrate 306 coated with an adhesive can be from about 150 µm to about 200 µm thick (e.g., about 175 µm to about 180 µm thick). Alternatively, a cyclo-olefin copolymer (COC)-based substrate (e.g., cycloolefin-copolymer TOPAS Type 8007S-04^{®}) can be used. Additionally, a substrate 306 can have similar polarity to polymers or polymer cocktails that can coat the substrate 306.

Referring to **FIG. 4****,** in some embodiments, an example sensor 400 can include an enzyme layer 302, a probe layer 304, and a substrate 306, as discussed above. Additionally, in some embodiments the sensor 400 can also include a protective layer 410. The protective layer 410 can 1) prevent or limit direct contact with the bodily fluid (e.g., blood, etc.), such as sieve, as barrier to limit interference with the enzyme or probe function, 2) provide anti-fouling properties, and/or 3) be a non-toxic interface between any of the sensors described herein (sensor 300, sensor 400, sensor 500, sensor 500, sensor 700) and the bodily fluid.

In some embodiments, the sensor 400 can also include at least one intermediate layer 420. The at least one separation layer 420 can be used to separate the enzyme layer 302 and probe layer 304. This can better bond the enzyme layer 302 and the probe layer 304, and/or can limit interferences between various solvents used in different layers. In some embodiments, the intermediate layer 420 can be a polyvinyl acetate (PVAc) layer, a cellulose acetate layer (CA), or a combination thereof. In some cases, the PVAc, CA, or combinations thereof can be dissolved in dimethylformamide (DMF)/H₂O.

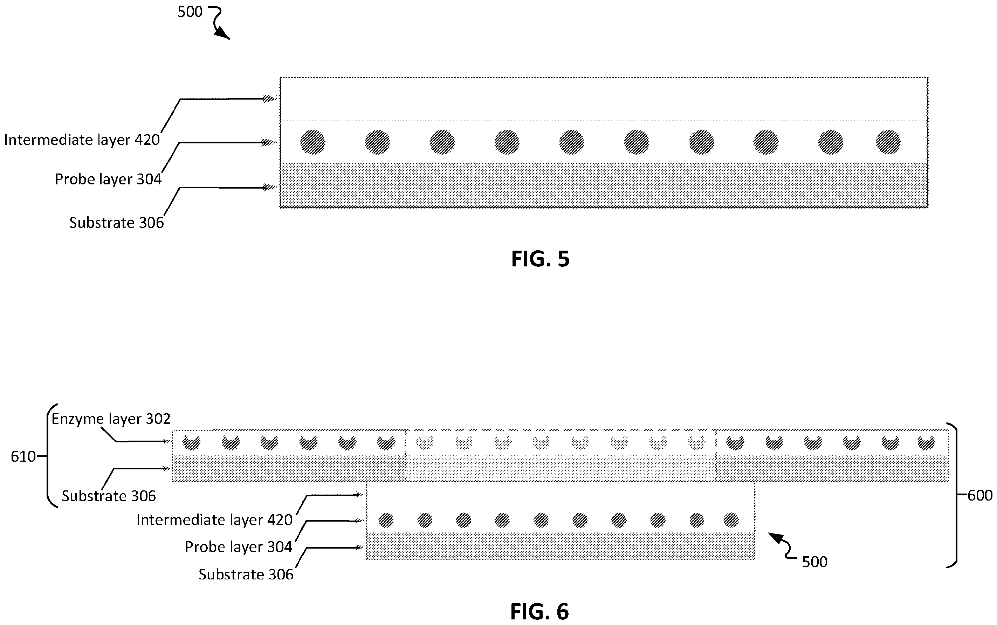

Referring to **FIG. 5****,** in some embodiments, any of the sensors described herein may use a probe construct 500 comprising an intermediate layer 420, a probe layer 304, and a substrate 306.

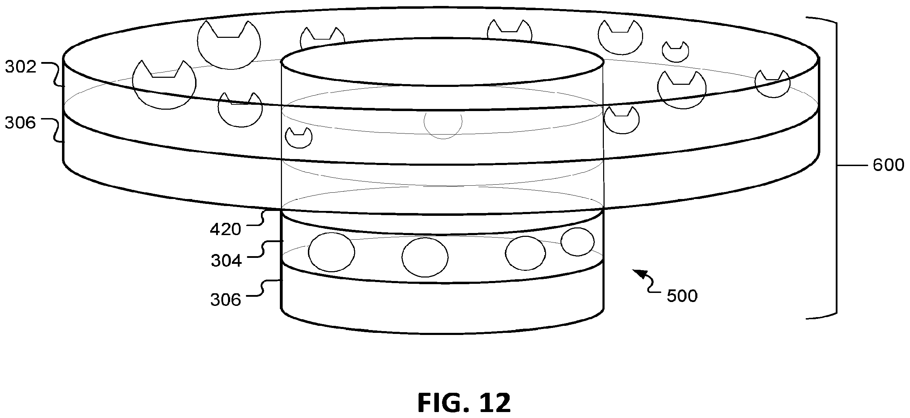

Referring to **FIG. 6** and **FIG. 12****,** in some embodiments, an example sensor 600 can include an enzyme layer 302 and a substrate 306 as an enzyme construct 610, and a probe construct 500, which includes an intermediate layer 420, a probe layer 304, and a substrate 306. The enzyme construct 610 can be an annular layer substantially centered on the probe construct 500. Such a construction creates a well-shaped sensor that defines an open interior space adjacent to the intermediate layer 420. The open interior space is surrounded by the substrate 306 and the enzyme layer 302. The enzyme construct 610 can be closer to the interior region than the probe construct 500. The enzyme layer 302 can be closer to the interior region than the substrate 306 of the enzyme construct 610. The probe layer 304 can be closer to the interior region than the substrate 306 of the probe construct 500.

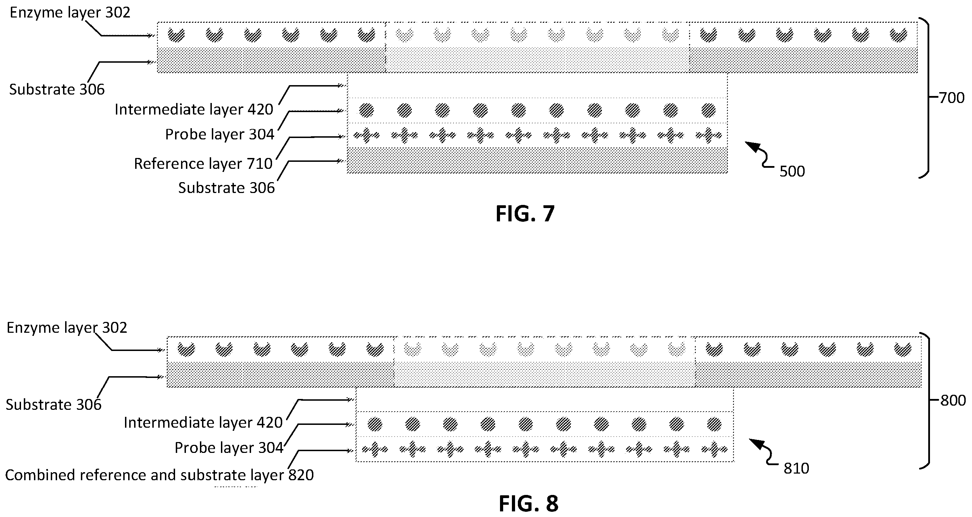

Referring to **FIG. 7****,** in some embodiments, another example sensor 700 can include an enzyme layer 302 and a substrate 306 as an enzyme construct 610, and a probe construct 500, which includes an intermediate layer 420, a probe layer 304, and a substrate 306, as discussed above. Additionally, in some embodiments the sensor 700 can include a reference layer 710. The reference layer 710 can be positioned farther from the open interior space than the probe layer 304.

In some embodiments, the reference layer 710 can include a reference dye. The reference layer 710 can be ratiometric. The reference dye can have an excitation maximum at 400±10 nm and an emission maximum at 450±10 nm. The reference dye can be selected from the group consisting of 9,10 di(phenylenthynyl)anthracene (DPEA), CD405M, 1-anilinonaphthalene-8-sulfonic acid, 8-benzyloxy-5,7-diphenylquinoline, 4-methylumbelliferyl acetate, octadecyl 7-hydroxycoumarine-3-carboxylate, perylene, tetracene, H9-40, pyranine, etyl eosin, coumarin 30, coumarin 153, and CF^{™}405M. Inclusion of a reference dye in any of the sensors described herein may prevent the need to calibrate the sensor with each use to produce accurate measurements. Any of the sensors described herein can include a reference layer 710, including, but not limited to, the sensor 400.

Referring to **FIG. 8****,** in some embodiments, another example sensor 800 can include an enzyme construct 610, as discussed above, and an alternative probe construct 810 with an intermediate layer 420, a combined reference and substrate layer 820. The combined reference and substrate layer 820 can be ratiometric. In some cases, the combined reference and substrate layer 820 includes 9,10 di(phenylenthynyl)anthracene (DPEA) and cyclo-olefin copolymer (COC).

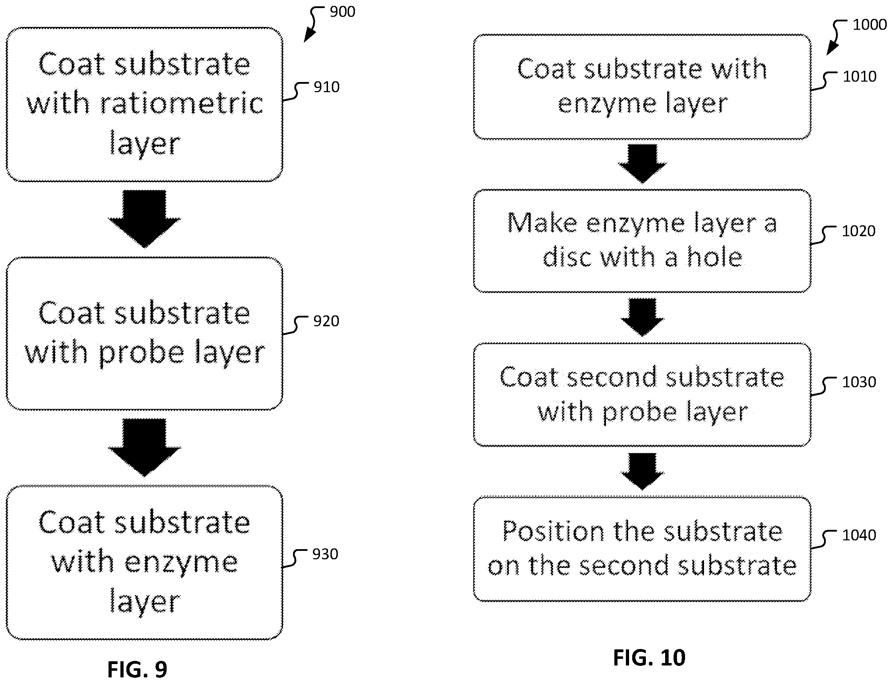

Referring to **FIG. 9****,** in some embodiments, the sensor 224 and/or any of the ratiometric sensors described herein can be manufactured using an example method 900. The method 900 can include a step 910 comprising coating the substrate with a ratiometric layer, a step 920 comprising coating the substrate with a probe layer, and a step 930 comprising coating the substrate with an enzyme layer. The substrates can be the same substrate or different substrates. Optionally, the layers, if they are on different substrates, can be joined with intermediate layers (e.g., intermediate layer 420), or held together physically.

Referring to **FIG. 10****,** in some embodiments, the sensor 224 and/or any of the sensors described herein with multiple substrates (e.g. sensor 600, sensor 700, or sensor 800) can be manufactured using an example method 1000. The method 1000 can include a step 1010 comprising coating the substrate 306 with an enzyme layer 302 to make an enzyme construct 610, a step 1020 comprising making the enzyme layer 302 a disc-shape defining a hole such that it is an annular layer defining an open interior space, a step 1030 comprising coating a second substrate with a probe layer 304 to make a probe construct 500, and a step 1040 comprising positioning the annular enzyme construct 610 on the probe construct 500.

Any of the coating of a substrate described herein can use knife coating and/or other suitable coating techniques. Multiple layers of a coating can be applied when appropriate.

Any of the sensors described herein can also include an intermediate layer (e.g., intermediate layer 420) between any of the enzyme layers described here in (e.g., enzyme layer 302) and any of the probe layers described herein (e.g., probe layer 304).

Any of the sensors described herein can be included in a blood parameter measurement device 220 and/or can be used in a HLM 100.

### EXAMPLES

### Example 1: Sensor 800 Preparation and Measurements in a Flow Cell

*Polymer Cocktail: 5 to 20 wt% D4 in EtOH*/*H₂O* - Hydromed D4 is dissolved in a mixture of ethanol and water (80-95% ethanol). Temperature is between about 20°C and about 50°C.

*Polymer Cocktail: 5 to 20 wt% PVAc*/*CA in cyanic acid (CHON)*/*H₂O -* CHON is mixed with water (95-99.9% CHON). CA and PVAc (approx. 95-99.9 wt% CA) are dissolved in CHON/H₂O. Temperature should be between 20°C and 50°C.

*Polymer Cocktail: 5 to 20 wt% PVAc*/*CA in DMF*/*H₂O* - DMF is mixed with water (80-95% DMF). CA and PVAc (approx. 95-99.9 wt% CA) are dissolved in DMF/H₂O. Temperature should be between 20°C and 50°C.

*Eu³' Stock Solution* - EuCl₃·6 H₂O dissolved in water and can be stored for at least 6 months under light protection at 4°C.

*DPEA Stock Solution* - DPEA is dissolved in toluene. The stock is stored in a glass vial with a sealed lid at 4°C and protected from light.

*Tetracycline Stock Solution* - Tetracycline HCl is dissolved in water. The solution must be used within 30 minutes and must be stored light protected.

*GOx and LOx Stock solution in 0.1 M HEPES pH 7.4* - GOx or LOx are dissolved in HEPES buffer under mild shaking for 10 to 20 min at 37°C. The solutions must be used immediately and cannot be stored.

*Eu-Tetracycline (EuTc) Stock Solution for Sensor Cocktail -* Tetracycline HCl is dissolved in the Eu³⁺ stock solution. The solution must be used immediately and cannot be stored.

*Sensor Cocktails* - D4 in ethanol/H₂O is mixed with the EuTc stock solution at ambient conditions. It is recommended to use the cocktail as soon as possible.

PVAc/CA in DMF/H₂O is mixed with EuTc stock solution at ambient conditions. It is recommended to use the cocktail as soon as possible.

*Enzyme Cocktails* - D4 in ethanol/H₂O and the enzyme stock solution are mixed at 37°C until a homogeneous suspension is obtained. It is recommended to use the cocktail immediately within 1 to 2 h.

*Reference Layer Cocktail* - Topas^{®} 8007S-04 polymer and 0.1-200 µL of a DPEA-stock in toluene are dissolved in toluene under light protection at room temperature.

### Sensor Foil Preparation

### Sensor layer: D4-EuTc//PVAc/CA

A homogeneous layer of the sensor cocktail with a wet thickness of 10 to 100 µm on a Mylar^{®} substrate is obtained through knife coating. After 30 seconds to 2 minutes, the sensor foils are moved from the coating device to the oven. The foils are dried for 3.0 ± 0.5 h at approx. 40 °C. The second layer is applied immediately after the drying time.

A homogeneous protection layer of the PVAc/CA cocktail with a wet thickness of 10 to 100 µm on the previously coated D4-EuTc sensor layer is obtained through knife coating. After 30 seconds to 2 minutes, the sensor foils are moved from the coating device to the oven. The foils are dried for 3.0 ± 0.5 h at approx. 40 °C.

The foils are washed for 15 ± 5 minutes in an excess of 0.1 M HEPES pH 7.4 to remove unbound complex and to rehydrate the hydrogel after drying.

The sheets can then be stacked carefully and stored light-protected in a closed zipper bag at 4°C until sensor disc preparation, or they can be used immediately for the next step. Sensor foils can be stored for at least 4 weeks under the above-mentioned conditions

### Sensor layer: PVAc/CA-EuTc

A homogeneous layer of the sensor cocktail with a wet thickness of 10 to 100 µm on a Mylar^{®} substrate is obtained through knife coating. After 30 seconds to 2 minutes, the sensor foils are moved from the coating device to the oven. The foils are dried for 3.0 ± 0.5 h at approx. 40 °C.

The foils are washed for 15 ± 5 minutes in an excess of 0.1 M HEPES pH 7.4 to remove unbound complex and to rehydrate the hydrogel after drying.

The sheets can then be stacked carefully and stored light-protected in a closed zipper bag at 4°C until sensor disc preparation, or they can be used immediately for the next step. Sensor foils can be stored for at least 4 weeks under the above-mentioned conditions.

### Enzyme Layer: D4-GOx/LOx

A homogeneous layer of the enzyme cocktail with a wet thickness of 10 to 100 µm on a Mylar^{®} substrate is obtained through knife coating. After 30 seconds to 2 minutes, the sensor foils are moved from the coating device to the oven. The foils are dried for 3.0 ± 0.5 h at approx. 40 °C.

The foils are washed for 15 ± 5 minutes in an excess of 0.1 M HEPES pH 7.4 to remove unbound enzyme and to rehydrate the hydrogel after drying.

The sheets can then be stacked carefully and stored light-protected in a closed zipper bag at 4°C until sensor disc preparation, or they can be used immediately for the next step. Enzyme foils can be stored for at least 6 months under the above-mentioned conditions.

The same procedure was used for all GOx concentrations and for LOx sensor foils.

### Reference Layer: DPEA-COC (Optional)

A homogeneous layer of the reference cocktail with a wet thickness of 10 to 100 µm on a Mylar^{®} substrate is obtained through knife coating. After 30 to 60 seconds, the sensor foils are moved from the coating device to the oven. The foils are dried for 4.0 ± 0.5 h at approx. 60 °C. The following layers can be applied later. The foils are stored at room temperature under light protection.

### Sensor Disc Preparation

### MTP Assays

For fixing the discs in the MTP double-adhesive tape is applied to the uncoated side of the Mylar^{®} support. Discs with a diameter of 6 mm are punched out of the foil with a toggle press. A distance to the edges of at least 2 mm is recommended. After removing the protective cover of the adhesive tape, the sensor discs are fixed on the bottom of the MTP wells. Usually, n = 4 or n = 8 discs are used for an assay. The diameter of the discs was selected due to geometry of the measurement device and can be adjusted accordingly.

### Flow Cell Assays

Discs with a diameter of 24 mm are punched out of the foil with a toggle press. A distance to the edges of at least 2 mm is recommended. The prepared discs can be stored light protected at 4°C for 4 weeks or can be used immediately. The diameter of the discs was selected due to geometry of the measurement device and can be adjusted accordingly.

### Measurement in a MTP Format

During all measurement, the samples are shaken orbitally by the plate reader. The samples are excited at 405/10 nm and the emission intensity is measured at 615/10 nm at regular interval at 25.0 ± 0.5 °C. Prior to the measurements the sensor discs are washed and rehydrated with HEPES buffer for 5 to 15 minutes. The respective glucose/HP samples (e.g., solutions of 0.5, 1, 2.5, 5, 10, 25 and 50 mM glucose/HP in buffer) are added to the sensor discs in the wells of the MTP. Kinetic measurements are started. The solution is removed before the next concentration is tested.

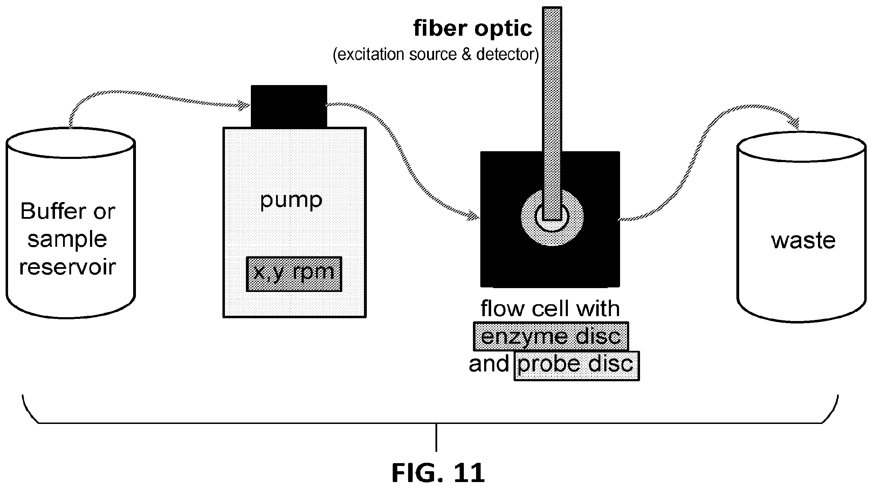

Experimental setup for detection of luminescent sensor discs in a flow cell. FIG. 11 is a schematic representation with two discs in one disc holder: for sample injection the pump is stopped, the intake-tube is put into the sample and the pump is started again for a certain time ("stop & go" method). FIG. 12 is a schematic drawing of the combination of sensor disc (inner part: EuTc-D4//PVAc/CA) with the enzyme disc (outer part: D4-GOx/LOx) for measurements.

The inlet tube is put in the buffer reservoir, the outlet tube to the waste and the pump is started at certain speed. The PMT voltage is set to a suitable level. Excitation wavelength is set to 405/8 nm. Emission intensity is detected at 615/8 nm. A time trace measurement with suitable data acquisition interval is started for a certain time. FIG. 11 shows the probe construct (D4-EuTc//PVAc/CA) is fixed in the middle of the flow cell surrounded by/ overlapping with an annular enzyme construct (D4-GOx). The constructs are held together via the pressure applied by the screws holding the flow cell together. The constructs can also be glued together by applying a solvent or glue on the parts where they overlap. After equilibration with HEPES buffer, the samples are injected.

While this specification contains many specific implementation details, these should not be construed as limitations, but rather as descriptions of features specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable subcombination.

Particular embodiments of the subject matter have been described. The invention is defined by the following claims. The processes depicted in the accompanying figures do not necessarily require the particular order shown, or sequential order, to achieve desirable results. In certain implementations, multitasking and parallel processing may be advantageous.

## Claims

1. A flow-through blood parameter measurement device (220) comprising:
a tubular housing (222) defining an open interior space configured for receiving blood and configured for the blood to flow through it; and
a sensor connected to the tubular housing, the sensor comprising:
i) a first layer (302) comprising a hydrogel in which an enzyme is trapped or immobilized, wherein the enzyme is configured to produce hydrogen peroxide when reacting with at least one biological analyte in the blood, and
ii) a second layer (304) comprising a substance that is chemically responsive to hydrogen peroxide, such that the at least one analyte can be monitored based on a readout of luminescent or fluorescent light produced by the substance, wherein the configuration is such that the first layer (302) is closer to the open interior space than the second layer (304).

2. The device of claim 1, wherein the enzyme is selected from the group consisting of glucose oxidase (GOx), lactate oxidase (LOx), cholesterol oxidase (ChOx), galactose oxidase (GAOx), pyruvate oxidase (POx), xanthine oxidase (XAOx), monoamine oxidase A (MAO-Ax), monoamine oxidase B (MAO-Bx), D-Amino acid oxidase (D-AAOx), L-Amino acid oxidase (L-AAOx), lactose oxidase (LOx), and superoxide dismutase (SOD).

3. The device of claim 1 or claim 2, wherein the enzyme comprises a glucose oxidase (GOx).

4. The device of any one of claims 1-3, wherein the enzyme comprises a lactate oxidase (LOx).

5. The device of any one of claims 1-4, wherein the substance comprises a Europium (III)-tetracycline (EuTu) complex.

6. The device of any one of claims 1-5, wherein the first layer and the second layer are directly adjacent to each other.

7. The device of any one of claims 1-6, wherein the sensor further comprises an intermediate layer (420) between the first layer and the second layer.

8. The device of any one of claims 1-6, wherein the first layer (320) is an annular layer and is substantially centered on the second layer (304).

9. The device of any one of claims 1-8, wherein the sensor further comprises a protective layer (410) positioned between the first layer and the open interior space.

10. The device of one of claims 1-9, wherein the sensor further comprises a reference dye, and
optionally wherein the reference dye has an excitation maximum at 400±10 nm and an emission maximum at 450±10 nm.

11. The device of claim 10, wherein the reference dye is selected from the group consisting of 9, 10 di(phenylenthynyl)anthracene (DPEA), 1-anilinonaphthalene-8-sulfonic acid, 8-benzyloxy-5,7-diphenylquinoline, 4-methylumbelliferyl acetate, octadecyl 7-hydroxycoumarine-3-carboxylate, perylene, tetracene, pyranine, etyl eosin, coumarin 30 and coumarin 153.

12. The device of any one of claims 10-11, wherein the sensor further comprises a reference layer (710, 820) comprising the reference dye, wherein the reference layer is positioned farther from the open interior space than the second layer (304).

13. The device of any preceding claim, further comprising:
a supply tube connected to one end of the blood parameter measurement device (220); and
a second tube connected the other end of the blood parameter measurement device (220) configured to flow blood away from the blood parameter measurement device (220).

14. The device of any preceding claim, wherein the open interior space is surrounded by the substrate (306) and the enzyme layer (302).

15. A heart-lung machine (HLM) system (100) comprising the device of any preceding claim.

## Patentansprüche

1. Durchfluss-Blutparametermessvorrichtung (220) umfassend:
ein rohrförmiges Gehäuse (222), das einen offenen Innenraum definiert, der zum Aufnehmen von Blut ausgelegt ist und dazu ausgelegt ist, dass das Blut hindurch fließt; und
einen Sensor, der mit dem rohrförmigen Gehäuse verbunden ist, wobei der Sensor umfasst:
i) eine erste Schicht (302), die ein Hydrogel umfasst, in dem ein Enzym eingeschlossen oder immobilisiert ist, wobei das Enzym dazu ausgelegt ist, Wasserstoffperoxid zu erzeugen, wenn es mit wenigstens einem biologischen Analyten in dem Blut reagiert, und
ii) eine zweite Schicht (304), die einen Stoff umfasst, der chemisch auf Wasserstoffperoxid anspricht, so dass der wenigstens eine Analyt auf der Grundlage einer Auslesung von Lumineszenz- oder Fluoreszenzlicht, das von dem Stoff erzeugt wird, überwacht werden kann, wobei die Konfiguration so ist, dass die erste Schicht (302) näher an dem offenen Innenraum als die zweite Schicht (304) liegt.

2. Vorrichtung nach Anspruch 1, wobei das Enzym ausgewählt ist aus der Gruppe bestehend aus Glucoseoxidase (GOx), Lactatoxidase (LOx), Cholesteroloxidase (ChOx), Galactoseoxidase (GAOx), Pyruvatoxidase (POx), Xanthinoxidase (XAOx), Monoaminoxidase A (MAO-Ax), Monoaminoxidase B (MAO-Bx), D-Aminosäureoxidase (D-AAOx), L-Aminosäureoxidase (L-AAOx), Lactoseoxidase (LOx) und Superoxiddismutase (SOD).

3. Vorrichtung nach Anspruch 1 oder Anspruch 2, wobei das Enzym eine Glucoseoxidase (GOx) umfasst.

4. Vorrichtung nach einem der Ansprüche 1-3, wobei das Enzym eine Lactatoxidase (LOx) umfasst.

5. Vorrichtung nach einem der Ansprüche 1-4, wobei der Stoff einen Europium(III)-Tetracyclin(EuTu)-Komplex umfasst.

6. Vorrichtung nach einem der Ansprüche 1-5, wobei die erste Schicht und die zweite Schicht direkt aneinander angrenzen.

7. Vorrichtung nach einem der Ansprüche 1-6, wobei der Sensor ferner eine Zwischenschicht (420) zwischen der ersten Schicht und der zweiten Schicht umfasst.

8. Vorrichtung nach einem der Ansprüche 1-6, wobei die erste Schicht (320) eine ringförmige Schicht ist und im Wesentlichen auf der zweiten Schicht (304) zentriert ist.

9. Vorrichtung nach einem der Ansprüche 1-8, wobei der Sensor ferner eine Schutzschicht (410) umfasst, die zwischen der ersten Schicht und dem offenen Innenraum angeordnet ist.

10. Vorrichtung nach einem der Ansprüche 1-9, wobei der Sensor ferner einen Referenzfarbstoff umfasst und gegebenenfalls wobei der Referenzfarbstoff ein Anregungsmaximum bei 400±10 nm und ein Emissionsmaximum bei 450±10 nm aufweist.

11. Vorrichtung nach Anspruch 10, wobei der Referenzfarbstoff ausgewählt ist aus der Gruppe bestehend aus 9,10-Di(phenylenthinyl)anthracen (DPEA), 1-Anilinonaphthalin-8-sulfonsäure, 8-Benzyloxy-5,7-diphenylchinolin, 4-Methylumbelliferylacetat, Octadecyl-7-hydroxycumarin-3-carboxylat, Perylen, Tetracen, Pyranin, Etyleosin, Cumarin 30 und Cumarin 153.

12. Vorrichtung nach einem der Ansprüche 10-11, wobei der Sensor ferner eine Referenzschicht (710, 820) umfasst, die den Referenzfarbstoff umfasst, wobei die Referenzschicht weiter von dem offenen Innenraum als die zweite Schicht (304) angeordnet ist.

13. Vorrichtung nach einem der vorstehenden Ansprüche, ferner umfassend:
ein Versorgungsrohr, das mit einem Ende der Blutparametermessvorrichtung (220) verbunden ist; und
ein zweites Rohr, das mit dem anderen Ende der Blutparametermessvorrichtung (220) verbunden ist und dazu ausgelegt ist, Blut von der Blutparametermessvorrichtung (220) weg zu leiten.

14. Vorrichtung nach einem der vorstehenden Ansprüche, wobei der offene Innenraum von dem Substrat (306) und der Enzymschicht (302) umgeben ist.

15. Herz-Lungen-Maschine(HLM)-System (100) umfassend die Vorrichtung nach einem der vorstehenden Ansprüche.

## Revendications

1. Dispositif de mesure de paramètres sanguins (220) à écoulement traversant comprenant :
un logement tubulaire (222) définissant un espace intérieur ouvert configuré pour recevoir le sang et configuré pour que le sang s'écoule à travers lui ; et
un capteur connecté au logement tubulaire, le capteur comprenant :
i) une première couche (302) comprenant un hydrogel dans lequel une enzyme est piégée ou immobilisée, l'enzyme étant configurée pour produire du peroxyde d'hydrogène lors de sa réaction avec au moins un analyte biologique dans le sang, et
ii) une seconde couche (304) comprenant une substance qui est chimiquement sensible au peroxyde d'hydrogène, de telle sorte que l'au moins un analyte puisse être surveillé sur la base d'une lecture d'une lumière luminescente ou fluorescente produite par la substance, la configuration étant telle que la première couche (302) est plus proche de l'espace intérieur ouvert que la seconde couche (304).

2. Dispositif selon la revendication 1, dans lequel l'enzyme est choisie dans le groupe constitué par glucose oxydase (GOx), lactate oxydase (LOx), cholestérol oxydase (ChOx), galactose oxydase (GAOx), pyruvate oxydase (POx), xanthine oxydase (XAOx), monoamine oxydase A (MAO-Ax), monoamine oxydase B (MAO-Bx), acide D-amino oxydase (D-AAOx), acide L-amino oxydase (L-AAOx), lactose oxydase (LOx), et superoxyde dismutase (SOD).

3. Dispositif selon la revendication 1 ou la revendication 2, dans lequel l'enzyme comprend une glucose oxydase (GOx).

4. Dispositif selon l'une quelconque des revendications 1 à 3, dans lequel l'enzyme comprend une lactate oxydase (LOx).

5. Dispositif selon l'une quelconque des revendications 1 à 4, dans lequel la substance comprend un complexe europium (III)-tétracycline (EuTu).

6. Dispositif selon l'une quelconque des revendications 1 à 5, dans lequel la première couche et la seconde couche sont directement adjacentes l'une à l'autre.

7. Dispositif selon l'une quelconque des revendications 1 à 6, dans lequel le capteur comprend en outre une couche intermédiaire (420) entre la première couche et la seconde couche.

8. Dispositif selon l'une quelconque des revendications 1 à 6, dans lequel la première couche (320) est une couche annulaire et est sensiblement centrée sur la seconde couche (304).

9. Dispositif selon l'une quelconque des revendications 1 à 8, dans lequel le capteur comprend en outre une couche protectrice (410) positionnée entre la première couche et l'espace intérieur ouvert.

10. Dispositif selon l'une des revendications 1 à 9, dans lequel le capteur comprend en outre un colorant de référence, et
éventuellement dans lequel le colorant de référence présente un maximum d'excitation à 400 ±10 nm et un maximum d'émission à 450 ±10 nm.

11. Dispositif selon la revendication 10, dans lequel le colorant de référence est choisi dans le groupe constitué par 9,10-bis(phénylènthynyl)anthracène (DPEA), acide 1-anilinonaphtalène-8-sulfonique, 8-benzyloxy - 5,7-diphénylquinoléine, acétate de 4-méthylumbelliféryle, 7-hydroxycoumarine-3-carboxylate d'octadécyle, pérylène, tétracène, pyranine, éthyléosine, coumarine 30 et coumarine 153.

12. Dispositif selon l'une quelconque des revendications 10 à 11, dans lequel le capteur comprend en outre une couche de référence (710, 820) comprenant le colorant de référence, dans lequel la couche de référence est positionnée plus loin de l'espace intérieur ouvert que la seconde couche (304).

13. Dispositif selon une quelconque revendication précédente, comprenant en outre :
un tube d'alimentation raccordé à une extrémité du dispositif de mesure de paramètres sanguins (220) ; et
un second tube raccordé à l'autre extrémité du dispositif de mesure de paramètres sanguins (220) configuré pour évacuer le sang du dispositif de mesure de paramètres sanguins (220).

14. Dispositif selon une quelconque revendication précédente, dans lequel l'espace intérieur ouvert est entouré par le substrat (306) et la couche enzymatique (302).

15. Système de machine cœur-poumon (HLM) (100) comprenant le dispositif selon une quelconque revendication précédente.
